(19) Europäisches Patentamt / European Patent Office / Office européen des brevets

(11) **EP 3 544 484 B1**

(12) **FASCICULE DE BREVET EUROPEEN**

(45) Date de publication et mention
de la délivrance du brevet:
**01.10.2025 Bulletin 2025/40**

(21) Numéro de dépôt: **17809247.4**

(22) Date de dépôt: **27.11.2017**

(51) Classification Internationale des Brevets (IPC):
***A61B 3/036*** (2006.01)   ***A61B 3/028*** (2006.01)
***G02C 13/00*** (2006.01)

(52) Classification Coopérative des Brevets (CPC):
**A61B 3/036;** A61B 3/028; G02C 13/005

(86) Numéro de dépôt international:
**PCT/EP2017/080499**

(87) Numéro de publication internationale:
**WO 2018/096140 (31.05.2018 Gazette 2018/22)**

(54) **PROCEDE ET DISPOSITIF DE BILAN INTERMEDIAIRE D'UN OEIL**

VERFAHREN UND VORRICHTUNG ZUR ZWISCHENBEURTEILUNG EINES AUGES

METHOD AND DEVICE FOR INTERMEDIATE ASSESSMENT OF AN EYE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **28.11.2016 FR 1661568**

(43) Date de publication de la demande:
**02.10.2019 Bulletin 2019/40**

(73) Titulaire: **SiVIEW
75014 Paris (FR)**

(72) Inventeur: **PICHEREAU-BASLE, Laure
91460 Marcoussis (FR)**

(74) Mandataire: **Cabinet Camus Lebkiri
25 rue de Maubeuge
75009 Paris (FR)**

(56) Documents cités:
**WO-A1-2016/084086     JP-A- 2011 013 373
US-A- 5 914 772     US-A1- 2006 152 675**

• **A-A A+ ET AL: "HOME ABOUT RESOURCES
NEWS & INFO PUBLICATIONS CONTACT
WRITINGS How Visual Acuity Is Measured
Posted in: Eye Conditions Share/Email/Print", 1
October 2003 (2003-10-01), XP055399156,
Retrieved from the Internet <URL:http://
lowvision.preventblindness.org/eye-conditions/
how-visual-acuity-is-measured/> [retrieved on
20170816]**

**Description**

**DOMAINE TECHNIQUE DE L'INVENTION**

Le domaine technique de l'invention est celui de la caractérisation de systèmes optiques. La présente invention concerne un procédé et un dispositif de bilan intermédiaire d'un œil pouvant en particulier être utilisé pour conduire ultérieurement un examen de vue.

**ARRIERE-PLAN TECHNOLOGIQUE DE L'INVENTION**

Un examen de vue est généralement réalisé sur un utilisateur pour déterminer s'il a besoin d'une correction, et le cas échéant, le type de cette correction. La correction est alors typiquement apportée via une paire de lunettes ou de lentilles de contact.

Le point de départ de l'examen de vue est une mesure de réfraction objective qui est généralement réalisée au moyen d'un autoréfractomètre ou qui peut être, à défaut, la précédente correction de l'utilisateur. Alternativement à l'utilisation d'un autoréfractomètre, une technique manuelle de skiascopie est parfois utilisée. La valeur de réfraction objective mesurée avec l'autoréfractomètre n'est pas directement utilisable pour une prescription pour les raisons suivantes :

- elle est souvent trop forte, surtout chez les sujets jeunes, avec une sur-évaluation en myopie et une sous-évaluation en hypermétropie ;
- elle fait souvent ressortir de faibles astigmatismes tensionnels qui ne doivent pas être prescrits ;
- elle ne permet pas de déterminer si le sujet voit net et de manière confortable avec sa correction ;
- dans certains cas rares, elle peut s'avérer complètement fausse.

L'examen de vue se poursuit donc avec une mesure de réfraction subjective, qui comprend les étapes suivantes :

1) étape monoculaire,
2) étape bioculaire,
3) étape binoculaire.

L'étape monoculaire comporte les étapes suivantes :

a) recherche de la valeur de myopie ou d'hypermétropie, dite « valeur de sphère au palier »,
b) recherche de la valeur d'astigmatisme, avec son axe et sa puissance,
c) si l'astigmatisme est modifié, vérification de la valeur de sphère précédemment obtenue.

L'étape monoculaire est réalisée d'abord pour un œil, puis pour l'autre œil. L'étape bioculaire et l'étape binoculaire sont chacune réalisées pour les deux yeux à la fois.

La réfraction subjective est réalisée par un praticien interagissant avec le sujet. Pour faire tester différentes corrections au sujet, le praticien utilise un matériel adapté, qui peut être :

- une paire de lunettes d'essai avec des verres d'essais,
- une tête de réfracteur manuelle,
- une tête de réfracteur automatique.

Dans le premier cas, le praticien change manuellement les verres d'essais de la paire de lunettes d'essais. Avec une tête de réfracteur, le praticien fait défiler différents verres devant les yeux du sujet, en utilisant des mollettes dans le cas d'une tête de réfracteur manuelle ou via une console de commande dans le cas d'une tête de réfracteur automatique.

Afin de réaliser des examens de vue de manière plus rapide que dans l'état de la technique, le brevet US 5914772 propose un procédé dans lequel la mesure de réfraction subjective est remplacée par des nouvelles étapes automatiques de mesure. Ce procédé se révèle toutefois moins précis et répétable que les procédés de l'état de la technique, notamment dans les cas d'astigmatisme.

**RESUME DE L'INVENTION**

L'invention offre une solution aux problèmes évoqués précédemment, en proposant un bilan intermédiaire pouvant être utilisé pour conduire ultérieurement un examen de vue de manière plus rapide que dans l'état de la technique, tout en étant au moins aussi précis et répétable.

Un premier aspect de l'invention concerne un procédé de bilan intermédiaire d'un œil d'un utilisateur tel que défini dans la revendication 1.

L'acuité visuelle d'un œil quantifie sa capacité à discerner un détail et se mesure au loin comme au près. L'acuité visuelle de l'œil se mesure en recherchant le plus petit angle possible sous lequel l'œil distingue ledit détail, qui est généralement une lettre, un caractère ou une figure que l'on appelle aussi optotype. L'optotype est placé à une distance donnée et ses dimensions sont progressivement diminuées. Par exemple, en vision de loin, l'optotype est placé en France à cinq mètres et dans les pays anglo-saxons à six mètres. Le plus petit angle possible sous lequel l'œil voit l'optotype est appelé angle minimum de résolution et l'acuité visuelle de l'œil est définie comme l'inverse de cet angle minimum de résolution. Plus l'œil est capable de distinguer des optotypes sous un petit angle, plus son acuité visuelle est grande.

Grâce au procédé selon le premier aspect de l'invention, on réalise un bilan intermédiaire permettant d'adapter et d'optimiser un bilan ultérieur complet. Le bilan préliminaire requiert d'abord un peu de temps supplémentaire mais permet ensuite une grande efficacité dans la réalisation du bilan ultérieur complet, en évitant de réaliser des manipulations ou des calculs qui n'aboutissent pas.

Le procédé de bilan intermédiaire d'un œil d'un utilisateur selon un aspect de l'invention ne peut pas remplacer une mesure de réfraction subjective d'un examen de vue. Il n'est pas nécessaire à la réalisation d'une prescription d'une correction visuelle ; il n'est pas non plus suffisant pour réaliser une prescription d'une correction visuelle.

Outre les caractéristiques qui viennent d'être évoquées dans le paragraphe précédent, le procédé de bilan intermédiaire d'un œil selon le premier aspect de l'invention peut présenter une ou plusieurs caractéristiques complémentaires parmi les suivantes, considérées individuellement ou selon toutes les combinaisons techniquement possibles :

- Après chaque acquisition d'une valeur ajustée AVA d'acuité visuelle de l'œil, si la valeur ajustée AVA d'acuité visuelle mesurée pour l'œil est supérieure ou égale à la valeur de référence AVR d'acuité visuelle, l'étape d'ajustement prend fin.

- Si, avec une optique ayant un jeu de paramètres dont la valeur de sphère présente une variation positive par rapport à la précédente valeur de sphère testée, ou dont la valeur d'axe présente une variation par rapport à la précédente valeur d'axe testée, ou dont les valeurs de cylindre et d'axe correspondent respectivement à une valeur totale attendue ATA, la valeur ajustée AVA d'acuité visuelle acquise pour l'œil est supérieure ou égale à la valeur de référence AVR d'acuité visuelle, alors le procédé comporte une étape selon laquelle on réalise une acquisition d'une valeur d'acuité visuelle de l'œil en augmentant de +0,75 dioptrie la valeur de sphère de l'optique.

- L'étape d'ajustement comporte une première sous-étape dans laquelle on distingue une catégorie de cylindre faible ou moyen et une catégorie de cylindre fort, une catégorie de sphère faible et une catégorie de sphère forte, et selon laquelle :

    o si le cylindre est fort, on réalise au plus quatre acquisitions d'une valeur ajustée AVA d'acuité visuelle de l'œil en faisant varier la valeur d'axe de cylindre de l'optique ;
    o si le cylindre est faible ou moyen et si la sphère est forte, on réalise au plus quatre acquisitions d'une valeur ajustée AVA d'acuité visuelle de l'œil en faisant varier la valeur de sphère de l'optique ;
    o si le cylindre est faible ou moyen et si la sphère est faible, on réalise au plus deux acquisitions d'une valeur ajustée AVA d'acuité visuelle de l'œil en faisant varier la valeur de sphère de l'optique.

- L'étape d'ajustement comporte une deuxième sous-étape telle que :

    o si le cylindre est faible, on réalise une comparaison du cylindre et de l'axe avec une valeur totale attendue ATA respectivement pour le cylindre et l'axe ; si le cylindre présente une différence de puissance supérieure ou égale à 0,75 dioptrie avec sa valeur totale attendue ATA et/ou si l'axe présente une différence supérieure ou égale à 25° avec sa valeur totale attendue ATA, alors on réalise une acquisition d'une valeur ajustée AVA d'acuité visuelle de l'œil en utilisant la valeur totale attendue ATA pour le cylindre et l'axe de l'optique ;
    o si le cylindre est moyen, on réalise au plus deux acquisitions d'une valeur ajustée AVA d'acuité visuelle de l'œil en faisant varier la valeur d'axe de l'optique ;
    o si le cylindre est fort et si la valeur de sphère SPH1 ou de sphère pondérée SPH1' est strictement supérieure à la valeur de cylindre CYL1, on réalise au plus quatre acquisitions de valeur ajustée AVA d'acuité visuelle de l'œil en faisant varier la valeur de sphère de l'optique ;
    o si le cylindre est fort et si la valeur de sphère SPH1 ou de sphère pondérée SPH1' est inférieure ou égale à la valeur de cylindre CYL1, on réalise une comparaison du cylindre et de l'axe avec la valeur totale attendue ATA pour le cylindre et l'axe ; si le cylindre présente une différence de puissance supérieure ou égale à 1,50 dioptries avec sa valeur attendue ATA et/ou si l'axe présente une différence supérieure ou égale à 20° avec sa valeur totale attendue ATA, alors on réalise une acquisition d'une valeur ajustée AVA d'acuité visuelle de l'œil en utilisant la valeur totale attendue ATA pour le cylindre et l'axe de l'optique.

- Si le cylindre est faible, l'étape d'ajustement est terminée à l'issue des première et deuxième sous-étapes, sinon l'étape d'ajustement comporte une troisième sous-étape telle que :

o si le cylindre est moyen, on réalise une comparaison du cylindre et de l'axe avec leur valeur totale attendue ATA ; si le cylindre présente une différence de puissance supérieure ou égale à 1,00 dioptrie avec sa valeur totale attendue ATA et/ou si l'axe présente une différence supérieure ou égale à 15° avec sa valeur totale attendue ATA, alors on réalise une acquisition d'une valeur ajustée AVA d'acuité visuelle de l'œil en utilisant la valeur totale attendue ATA pour le cylindre et l'axe de l'optique ;
o si le cylindre est fort et si la valeur de sphère SPH1 ou de sphère pondérée SPH1' est strictement supérieure à la valeur de cylindre CYL1, on réalise une comparaison du cylindre et de l'axe avec leur valeur totale attendue ATA ; si le cylindre présente une différence de puissance supérieure ou égale à 1,50 dioptries avec sa valeur totale attendue ATA et/ou si l'axe présente une différence supérieure ou égale à 20° avec sa valeur totale attendue ATA, alors on réalise une acquisition d'une valeur ajustée AVA d'acuité visuelle de l'œil en utilisant la valeur totale attendue ATA pour le cylindre et l'axe de l'optique ;
o si le cylindre est fort et si la valeur de sphère SPH1 ou de sphère pondérée SPH1' est inférieure ou égale à la valeur de cylindre CYL1, on réalise au plus quatre acquisitions de valeur ajustée AVA d'acuité visuelle de l'œil en faisant varier la valeur de sphère de l'optique.

Un deuxième aspect de l'invention concerne un produit programme d'ordinateur comportant des instructions logicielles qui, lorsque le programme est exécuté par un ordinateur, mettent en œuvre le procédé selon le premier aspect de l'invention. Le programme d'ordinateur comprenant des instructions exécutables par une machine pour mettre en œuvre le procédé selon le premier aspect de l'invention peut être mis en œuvre par un ordinateur comprenant au moins une interface, un processeur et une mémoire physique non transitoire, également désignée d'une manière générale comme étant un support non transitoire lisible par ordinateur ou une mémoire non transitoire de stockage. L'ordinateur est un ordinateur à usage particulier, étant donné qu'il est programmé pour exécuter les étapes spécifiques du procédé décrit dans le présent document. La mémoire non transitoire est codée ou programmée avec des instructions de code spécifiques pour mettre en œuvre le procédé décrit dans le présent document et les étapes qui lui sont associées. La mémoire non transitoire communique avec le processeur physique de sorte que le processeur physique, lorsqu'il est utilisé, lit et exécute les instructions de code spécifiques qui sont intégrées dans la mémoire non transitoire. L'interface de l'ordinateur à usage particulier communique avec le processeur physique et reçoit des paramètres d'entrée qui sont traités par le processeur physique.

Un troisième aspect de l'invention concerne un support d'enregistrement lisible par un ordinateur, sur lequel est enregistré le produit programme d'ordinateur selon le deuxième aspect de l'invention. Diverses formes de supports d'enregistrement lisibles par ordinateur peuvent être utilisées pour l'exécution d'une ou plusieurs séquences d'une ou plusieurs instructions au processeur. Le terme « support d'enregistrement lisible par ordinateur », tel qu'utilisé ici, fait référence à tout support participant à fournir des instructions à un processeur pour leur l'exécution desdites instructions. Un tel support peut prendre de nombreuses formes, y compris, de manière non limitative : des supports non volatils, des supports volatils et des supports de transmission. Les supports non volatils comprennent, par exemple, des disques optiques ou magnétiques. Les supports volatils comprennent, par exemple, des mémoires dynamiques. Les supports de transmission comprennent, par exemple, des câbles coaxiaux, des fils de cuivre et des fibres optiques. Les formes courantes de supports lisibles par ordinateur comprennent, par exemple, une disquette, un disque souple, un disque dur, une bande magnétique ou tout autre support magnétique, un CD-ROM, un DVD ou tout autre support optique, des cartes perforées, bandes de papier ou tout autre support physique avec des motifs de trou, une RAM, une PROM, une EPROM, une mémoire FLASH-EPROM ou tout autre puce ou cartouche mémoire, une onde porteuse, et tout autre support à partir duquel un ordinateur peut lire.

Un quatrième aspect de l'invention concerne un dispositif de bilan intermédiaire d'un œil comportant :

- une mémoire de stockage d'une matrice ayant une pluralité de L lignes et une pluralité de C colonnes et comportant donc $L \times C$ composantes, chaque ligne correspondant à une catégorie de jeu de paramètres, chaque colonne correspondant à une catégorie d'âge et chaque composante étant associée à une valeur de référence AVR d'acuité visuelle,
- une mémoire de stockage d'un jeu de paramètres comportant une valeur de sphère, une valeur de cylindre et une valeur d'axe de cylindre,
- une pluralité d'optiques,

- un moyen pour agencer une optique de la pluralité d'optiques devant l'œil,
- un moyen d'affichage,
- un moyen d'acquisition de valeurs d'acuité visuelle de l'œil,
- une mémoire de stockage d'une valeur d'acuité visuelle acquise,
- un calculateur comportant des moyens pour la réalisation des étapes du procédé de bilan intermédiaire d'un œil selon le premier aspect de l'invention.

Chaque optique de la pluralité d'optiques a, par rapport au jeu initial de paramètres M1 de l'œil, un jeu ajusté de paramètres M2 comportant une valeur de sphère SPH2, une valeur de cylindre CYL2 et une valeur d'axe de cylindre AX2. Le jeu ajusté de paramètres M2 évolue à chaque ajustement. Les optiques de la pluralité d'optiques n'ont donc pas toutes le même jeu ajusté de paramètres M2.

L'invention et ses différentes applications seront mieux comprises à la lecture de la description qui suit et à l'examen des figures qui l'accompagnent.

## BREVE DESCRIPTION DES FIGURES

Les figures sont présentées à titre indicatif et nullement limitatif de l'invention.

- La figure 1 montre une représentation schématique d'un procédé de bilan intermédiaire d'un œil selon un mode de réalisation de l'invention.
- La figure 2a montre une première représentation schématique des sous-étapes d'une étape d'ajustement du bilan intermédiaire de la figure 1a.
- La figure 2b montre une deuxième représentation schématique, en détails, des sous-étapes de l'étape d'ajustement de la figure 2a.
- La figure 2c montre une représentation schématique d'une étape de test réalisée après chaque acquisition d'une valeur ajustée d'acuité visuelle, au cours de l'étape d'ajustement des figures 2a et 2b.
- La figure 3 montre une représentation schématique d'un procédé comportant le bilan intermédiaire de la figure 1a suivi d'un bilan complet.

## DESCRIPTION DETAILLEE D'AU MOINS UN MODE DE REALISATION DE L'INVENTION

Sauf précision contraire, un même élément apparaissant sur des figures différentes présente une référence unique.

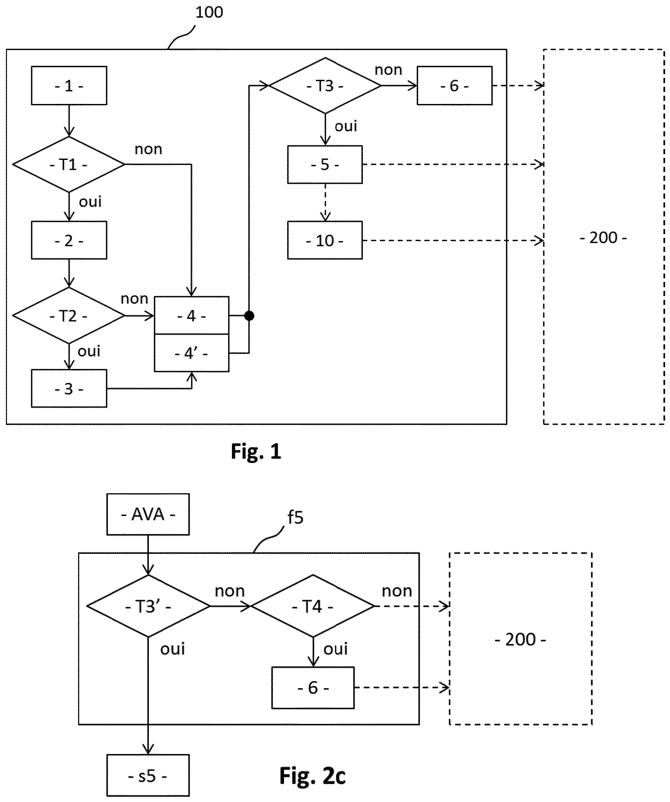

La figure 1 montre une représentation schématique d'un procédé 100 de bilan intermédiaire d'un œil selon un mode de réalisation de l'invention. Le procédé 100 est préférentiellement mis en œuvre au moyen d'un dispositif (non représenté) de bilan intermédiaire comprenant :

- une première mémoire de stockage d'une matrice ayant une pluralité de L lignes et une pluralité de C colonnes et comportant donc $L \times C$ composantes, chaque ligne correspondant à une catégorie de jeu de paramètres, chaque colonne correspondant à une catégorie d'âge et chaque composante étant associée à une valeur de référence AVR d'acuité visuelle,
- une deuxième mémoire de stockage d'une donnée d'âge et d'un jeu de paramètres comportant une valeur de sphère, une valeur de cylindre et une valeur d'axe de cylindre,
- une pluralité d'optiques,
- un moyen pour agencer une optique d'une pluralité d'optiques devant l'œil: ce moyen est préférentiellement une tête de réfracteur automatique, mais peut alternativement être une tête de réfracteur manuelle ou une paire de lunettes d'essais avec des verres d'essais,
- un moyen d'affichage en vision de loin, comportant de préférence un écran, polarisé ou non, ou alternativement un projecteur ou une échelle cartonnée ;
- un moyen d'acquisition de valeurs d'acuité visuelle de l'œil,
- une troisième mémoire de stockage d'une valeur d'acuité visuelle acquise,
- un calculateur comportant des moyens pour la réalisation des étapes du procédé 100 de bilan intermédiaire d'un œil.

Les première, deuxième et troisième mémoires de stockage peuvent être des première, deuxième et troisième parties d'une même mémoire de stockage. Alternativement, les première, deuxième et troisième mémoires de stockages peuvent former chacune une mémoire de stockage distincte.

Le procédé 100 comprend une étape 1 selon laquelle le calculateur attribue une valeur de référence AVR d'acuité visuelle à l'œil. Le calculateur utilise pour ce faire une donnée d'âge de l'œil et un jeu initial de paramètres M1 comportant :

- une valeur initiale de sphère SPH1,
- une valeur initiale de cylindre CYL1 et
- une valeur initiale d'axe de cylindre AX1.

La donnée d'âge et/ou le jeu initial de paramètres peuvent être fournis au dispositif de bilan intermédiaire via :

- une mesure objective, ou
- une saisie via une interface homme-machine telle qu'un clavier, ou
- une lecture de la deuxième mémoire de stockage.

Une mesure objective de réfraction via un autoréfractomètre ou un autoréfractokératomètre peut permettre d'obtenir le jeu initial de paramètres. Alternativement, un jeu initial de paramètres correspondant à une ancienne prescription peut être saisi. Selon une autre alternative, un jeu de paramètres résultant d'un précédent bilan intermédiaire et enregistré dans la deuxième mémoire de stockage peut être lu et utilisé comme jeu initial de paramètres lors d'un nouveau bilan intermédiaire.

Le procédé 100 comprend ensuite une étape de test T1 selon laquelle le calculateur répond à la question suivante : la valeur initiale de sphère SPH1 est-elle telle que :

$$-5,00 \; dioptries \; \leq SPH1 \leq +3,00 \; dioptries$$

?

- Si non, l'étape suivante du procédé 100 est une étape 4 selon laquelle une valeur modifiée AVM d'acuité visuelle de l'œil est acquise, en agençant devant l'œil une optique ayant le jeu initial de paramètres M1.
- Si oui, l'étape suivante du procédé 100 est une étape 2 selon laquelle une valeur brute AVB d'acuité visuelle de l'œil est acquise. Aucune optique n'est placée devant l'œil lors de l'étape 2.

L'étape 2 est suivie d'une étape de test T2 selon laquelle le calculateur répond à la question suivante : la valeur brute AVB d'acuité visuelle est-elle strictement supérieure à une valeur brute de référence AVBR d'acuité visuelle ? La valeur brute de référence AVBR d'acuité visuelle est une valeur brute de référence AVBR d'acuité visuelle qui est par exemple obtenue à partir du jeu initial de paramètres M1 et d'une règle connue appelée règle de Swaine.

- Si la valeur brute AVB d'acuité visuelle est inférieure ou égale à la valeur brute de référence AVBR d'acuité visuelle, l'étape suivante du procédé 100 est l'étape 4 précédemment décrite.
- Si la valeur brute AVB d'acuité visuelle est strictement supérieure à la valeur brute de référence AVBR d'acuité visuelle, l'étape suivante du procédé 100 est une étape 3 selon laquelle le calculateur pondère le jeu initial de paramètres M1 pour l'obtention d'un jeu pondéré de paramètres M1' ayant la valeur initiale de cylindre CYL1, la valeur initiale d'axe de cylindre AX1 et une valeur pondérée de sphère SPH1' telle que :

$$SPH1 + 0,25 \; dioptrie \leq SPH1' \leq SPH1 + 0,75 \; dioptrie$$

L'étape 3 est suivie d'une étape 4' selon laquelle une valeur modifiée AVM' d'acuité visuelle de l'œil est acquise, en agençant devant l'œil une optique ayant le jeu pondéré de paramètres M1'.

L'étape 4 ou l'étape 4' sont suivies d'une étape T3 de test selon laquelle le calculateur répond à la question suivante : la valeur modifiée AVM, AVM' d'acuité visuelle de l'œil est-elle strictement inférieure à la valeur de référence AVR d'acuité visuelle de l'œil ?

- Si la réponse est non, le procédé 100 de bilan intermédiaire comporte préférentiellement une étape 6 selon laquelle on réalise une acquisition d'une valeur d'acuité visuelle de l'œil en augmentant de +0,75 dioptrie la valeur de sphère de départ SPH1, SPH1' de l'optique. L'étape 6 correspond à une vérification d'accommodation, puis le procédé 100 de bilan intermédiaire est terminé. Il est préférentiellement suivi d'un procédé 200 de bilan complet, ultérieurement décrit.
- Si la réponse est oui, l'étape suivante du procédé 100 est une étape 5 d'ajustement selon laquelle au moins une valeur ajustée AVA d'acuité visuelle de l'œil est acquise, en agençant devant l'œil une optique ayant un jeu ajusté de paramètres M2. Chaque ajustement utilise comme point de départ le jeu initial de paramètres M1 (SPH1, CYL1, AX1) ou, si l'étape 3 de pondération a eu lieu, le jeu pondéré de paramètres M1'(SPH1', CYL1, AX1). Dans la description détaillée de l'étape 5 d'ajustement, ci-après, on utilise l'expression « valeur de sphère de départ SPH1, SPH1' » pour désigner la valeur initiale de sphère SPH1 ou, si l'étape 3 de pondération a eu lieu, la valeur pondérée de sphère SPH1'.

La figure 2c montre une représentation schématique d'une étape f5 qui est avantageusement réalisée après chaque acquisition d'une valeur ajustée AVA d'acuité visuelle de l'œil, au cours de l'étape 5 d'ajustement (référence « AVA » sur la figure 2c). L'étape f5 comporte une sous-étape de test T3' selon laquelle le calculateur répond à la question : la valeur ajustée AVA d'acuité visuelle de l'œil est-elle strictement inférieure à la valeur de référence AVR d'acuité visuelle de l'œil ?

- Si la réponse est oui, l'étape 5 d'ajustement se poursuit (référence « s5 » sur la figure 2c).
- Si la réponse est non, l'étape 5 d'ajustement est terminée. Selon un mode de réalisation, l'étape f5 et le procédé 100 de bilan intermédiaire sont également terminés. Selon un autre mode de réalisation, l'étape f5 comporte avantageusement une sous-étape de test T4 selon laquelle le calculateur répond à la question : la valeur de sphère de l'optique présente-t-elle une variation positive par rapport à la valeur de sphère de départ SPH1, SPH1', ou la valeur d'axe de l'optique présente-t-elle une variation par rapport à la valeur d'axe initiale AX1, ou les valeurs de cylindre et d'axe de l'optique correspondent-elles respectivement à une valeur totale attendue ATA ?

  o Si la réponse est oui, l'étape f5 comporte l'étape 6 précédemment décrite, selon laquelle on réalise une acquisition d'une valeur d'acuité visuelle de l'œil en augmentant de +0,75 dioptrie la valeur de sphère de départ SPH1, SPH1' de l'optique. On note que l'étape 6 est réalisée systématiquement à l'issue du test T4, sauf lorsque la valeur de sphère de l'optique présente une variation négative par rapport à la valeur de sphère de départ SPH1, SPH1'. L'étape 6 correspond à une vérification d'accommodation. L'étape f5 et le procédé 100 de bilan intermédiaire sont ensuite terminés.
  o Si la réponse est non, l'étape f5 et le procédé 100 de bilan intermédiaire sont terminés.

L'étape f5 permet d'interrompre le procédé 100 de bilan intermédiaire dès que la valeur ajustée AVA d'acuité visuelle de l'œil est supérieure ou égale à la valeur de référence AVR.

Si la valeur de référence AVR d'acuité visuelle n'a pas été atteinte à l'issue de l'étape 5 d'ajustement, le procédé 100 de bilan intermédiaire se poursuit avantageusement avec une étape 10 selon laquelle on réalise une acquisition d'une valeur d'acuité visuelle de l'œil en agençant devant l'œil une optique qui est un trou sténopéïque. Le procédé 100 de bilan intermédiaire est ensuite terminé. Il est avantageusement suivi d'un procédé 200 de bilan complet, ultérieurement décrit.

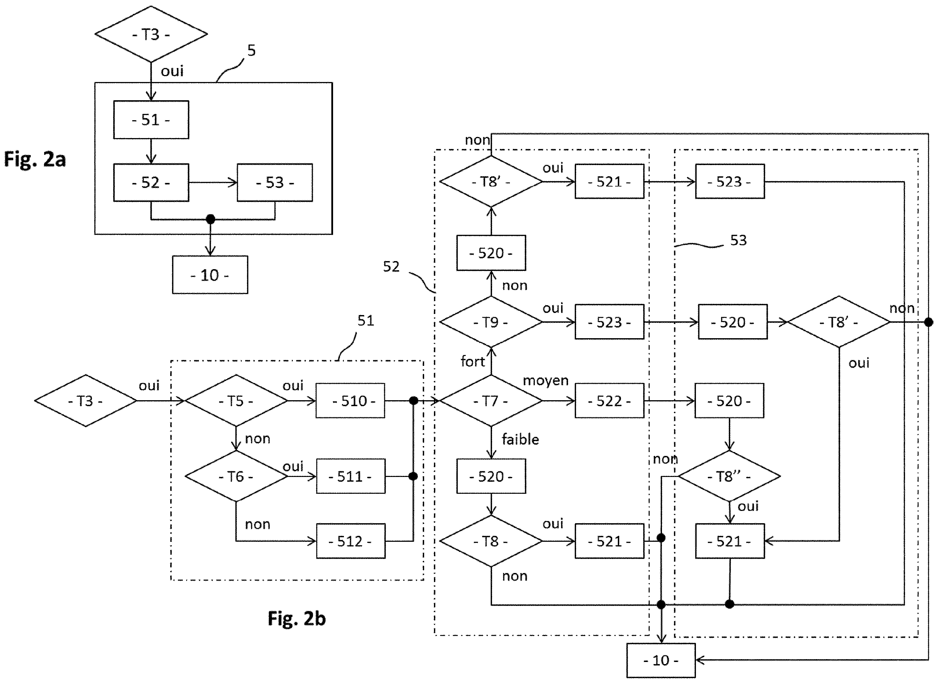

L'étape 5 d'ajustement est à présent décrite de manière détaillée, en lien avec les figures 2a et 2b. La figure 2a montre une première représentation schématique des sous-étapes d'une étape d'ajustement du bilan intermédiaire de la figure 1a. La figure 2b montre une deuxième représentation schématique, en détails, des sous-étapes de l'étape d'ajustement de la figure 2a.

L'étape 5 d'ajustement comporte une première sous-étape 51 suivie d'une deuxième sous-étape 52. A l'issue de la deuxième sous-étape 52 :

- soit l'étape 5 d'ajustement est terminée,
- soit l'étape 5 d'ajustement se poursuit avec une troisième sous-étape 53, puis l'étape 5 d'ajustement est terminée.

La première sous-étape 51 comporte préférentiellement un test T5 selon lequel le calculateur répond à la question : la valeur initiale de cylindre CYL1 est-elle forte ?

- Si la réponse est oui, on réalise une étape 510 selon laquelle au plus quatre acquisitions d'une valeur ajustée AVA d'acuité visuelle de l'œil sont réalisées, en faisant varier la valeur d'axe de cylindre de l'optique par rapport à la valeur initiale AX1. La première sous-étape 51 est ensuite terminée.
- Si la réponse est non, on réalise un test T6 selon lequel le calculateur répond à la question : la valeur de sphère de départ SPH1, SPH1' est-elle forte ?

  o Si la réponse est oui, on réalise une étape 511 selon laquelle au plus quatre acquisitions d'une valeur ajustée AVA d'acuité visuelle de l'œil sont réalisées, en faisant varier la valeur de sphère de l'optique par rapport à la valeur de sphère de départ SPH1, SPH1'. La première sous-étape 51 est ensuite terminée.
  o Si la réponse est non, on réalise une étape 512 selon laquelle au plus deux acquisitions d'une valeur ajustée AVA d'acuité visuelle de l'œil sont réalisées, en faisant varier la valeur de sphère de l'optique par rapport à la valeur de sphère de départ SPH1, SPH1'. La première sous-étape 51 est ensuite terminée.

Chaque acquisition réalisée lors de l'étape 510 utilise un jeu ajusté de paramètres M2 pour l'optique, ayant :

- une valeur de sphère SPH2 égale à la valeur de sphère de départ SPH1, SPH1', et
- une valeur de cylindre CYL2 égale à la valeur initiale de cylindre CYL1.

Lors de la première acquisition de l'étape 510, la valeur d'axe de cylindre AX2 du jeu ajusté de paramètres M2 présente préférentiellement une variation de +5° par rapport à la valeur initiale d'axe de cylindre AX1 :

$$AX2 = AX1 + 5°$$

L'étape f5 précédemment décrite a ensuite lieu, qui détermine si l'étape 5 d'ajustement continue ou est terminée. Si l'étape 5 d'ajustement continue, une deuxième acquisition de l'étape 510 est réalisée avec une valeur d'axe de cylindre AX2 du jeu ajusté de paramètres M2 qui présente préférentiellement une variation de -5° par rapport à la valeur initiale d'axe de cylindre AX1 :

$$AX2 = AX1 - 5°$$

L'étape f5 a ensuite lieu. Si l'étape 5 d'ajustement continue à l'issue de l'étape f5, une troisième acquisition de l'étape 510 est réalisée avec une valeur d'axe de cylindre AX2 du jeu ajusté de paramètres M2 qui présente préférentiellement une variation de +15° par rapport à la valeur initiale d'axe de cylindre AX1 :

$$AX2 = AX1 + 15°$$

L'étape f5 a ensuite lieu. Si l'étape 5 d'ajustement continue à l'issue de l'étape f5, une quatrième acquisition de l'étape 510 est réalisée avec une valeur d'axe de cylindre AX2 du jeu ajusté de paramètres M2 qui présente préférentiellement une variation de -15° par rapport à la valeur initiale d'axe de cylindre AX1 :

$$AX2 = AX1 - 15°$$

L'étape f5 a ensuite lieu. Si l'étape 5 d'ajustement n'est pas terminée à l'issue de l'étape f5, l'étape 5 d'ajustement continue avec la deuxième sous-étape 52.

Chaque acquisition réalisée lors de l'étape 511 utilise un jeu ajusté de paramètres M2 pour l'optique, ayant :

- une valeur de cylindre CYL2 égale à la valeur initiale de cylindre CYL1, et
- une valeur d'axe de cylindre AX2 égale à la valeur initiale d'axe de cylindre AX1.

Lors de la première acquisition de l'étape 511, la valeur de sphère SPH2 du jeu ajusté de paramètres M2 présente préférentiellement une première variation positive par rapport à la valeur de sphère de départ SPH1, SPH1' :

$$SPH2 = (SPH1, SPH1') + k1\ dioptrie$$

L'étape f5 précédemment décrite a ensuite lieu, qui détermine si l'étape 5 d'ajustement continue ou est terminée. Si l'étape 5 d'ajustement continue, une deuxième acquisition de l'étape 511 est réalisée avec une valeur de sphère SPH2 du jeu ajusté de paramètres M2 qui présente préférentiellement une première variation négative par rapport à la valeur de sphère de départ SPH1, SPH1' :

$$SPH2 = (SPH1, SPH1') - k1\ dioptrie$$

L'étape f5 a ensuite lieu. Si l'étape 5 d'ajustement continue à l'issue de l'étape f5, une troisième acquisition de l'étape 511 est réalisée avec une valeur de sphère SPH2 du jeu ajusté de paramètres M2 qui présente préférentiellement une deuxième variation positive par rapport à la valeur de sphère de départ SPH1, SPH1' :

$$SPH2 = (SPH1, SPH1') + k2\ dioptrie$$

L'étape f5 a ensuite lieu. Si l'étape 5 d'ajustement continue à l'issue de l'étape f5, une quatrième acquisition de l'étape 511 est réalisée avec une valeur de sphère SPH2 du jeu ajusté de paramètres M2 qui présente préférentiellement une deuxième variation négative par rapport à la valeur de sphère de départ SPH1, SPH1' :

$$SPH2 = (SPH1, SPH1') - k2\ dioptrie$$

L'étape f5 a ensuite lieu. Si l'étape 5 d'ajustement n'est pas terminée à l'issue de l'étape f5, l'étape 5 d'ajustement continue avec la deuxième sous-étape 52.

Lors de l'étape 511, les valeurs k1 et k2 des première et deuxième variations sont préférentiellement choisies parmi (0,50 ; 1,00 ; 1,50).

Chaque acquisition réalisée lors de l'étape 512 utilise un jeu ajusté de paramètres M2 pour l'optique, ayant :

- une valeur de cylindre CYL2 égale à la valeur initiale de cylindre CYL1, et
- une valeur d'axe de cylindre AX2 égale à la valeur initiale d'axe de cylindre AX1.

Lors de la première acquisition de l'étape 512, la valeur de sphère SPH2 du jeu ajusté de paramètres M2 présente préférentiellement une variation positive par rapport à la valeur de sphère de départ SPH1, SPH1' :

$$SPH2 = (SPH1, SPH1') + 0,50\ dioptrie$$

L'étape f5 précédemment décrite a ensuite lieu, qui détermine si l'étape 5 d'ajustement continue ou est terminée. Si l'étape 5 d'ajustement continue, une deuxième acquisition de l'étape 512 est réalisée avec une valeur de sphère SPH2 du jeu ajusté de paramètres M2 qui présente préférentiellement une variation négative par rapport à la valeur de sphère de départ SPH1, SPH1' :

$$SPH2 = (SPH1, SPH1') - 0,50\ dioptrie$$

L'étape f5 a ensuite lieu. Si l'étape 5 d'ajustement n'est pas terminée à l'issue de l'étape f5, l'étape 5 d'ajustement continue avec la deuxième sous-étape 52.

La deuxième sous-étape 52 comporte préférentiellement un test T7 selon lequel le calculateur répond à la question : la valeur initiale de cylindre CYL1, est-elle faible, moyenne ou forte ?

- Si la réponse est « faible », on réalise une étape 520 selon laquelle on compare la valeur initiale de cylindre CYL1 et la valeur initiale d'axe de cylindre AX1 avec une valeur totale attendue ATA pour ces deux valeurs. On réalise ensuite un test T8 selon lequel le calculateur répond à la question : « la valeur initiale de cylindre CYL1 présente-t-elle une différence de puissance supérieure ou égale à x dioptrie avec sa valeur totale attendue ATA, et/ou la valeur initiale d'axe de cylindre AX1 présente-t-elle une différence supérieure ou égale à y° avec sa valeur totale attendue ATA ? ». Dans le cadre du critère appliqué lors du test T8, la valeur de x dioptrie appartient préférentiellement à l'intervalle [0,75 dioptrie ; 1,25 dioptrie] et est plus préférentiellement égale à 0,75 dioptrie. La valeur de y° appartient préférentiellement à l'intervalle [20° ; 25°] et est plus préférentiellement égale à 25°.

   o Si la réponse est non, la deuxième sous-étape 52 et l'étape 5 d'ajustement sont terminées. Le procédé 100 se poursuit avec l'étape 10 précédemment décrite.
   o Si la réponse est oui, on réalise une étape 521 selon laquelle on réalise une acquisition d'une valeur ajustée AVA d'acuité visuelle de l'œil, en utilisant la valeur totale attendue ATA pour le cylindre et l'axe de l'optique. On réalise ensuite l'étape f5 précédemment décrite. Dans ce cas particulier, si l'étape 5 d'ajustement ne prend pas fin au cours de l'étape f5, la suite « s5 » de l'étape 5 d'ajustement a lieu mais correspond également à la fin de l'étape 5 d'ajustement, puis le procédé 100 de bilan intermédiaire se poursuit avec l'étape 10 précédemment décrite.

- Si la réponse est « moyenne », on réalise une étape 522 selon laquelle au plus deux acquisitions d'une valeur ajustée AVA d'acuité visuelle de l'œil sont réalisées, en faisant varier la valeur d'axe de l'optique par rapport à la valeur initiale d'axe de cylindre AX1. La deuxième sous-étape 52 est ensuite terminée et l'étape 5 d'ajustement se poursuit avec la troisième sous-étape 53.
- Si la réponse est « forte », on réalise un test T9 selon lequel le calculateur répond à la question : la valeur de sphère de départ SPH1, SPH1' est-elle strictement supérieure à la valeur initiale de cylindre CYL1 ?

   o Si la réponse est oui, on réalise une étape 523 selon laquelle au plus quatre acquisitions de valeur ajustée AVA d'acuité visuelle de l'œil sont réalisées, en faisant varier la valeur de sphère de l'optique par rapport à la valeur de sphère de départ SPH1, SPH1'. La deuxième sous-étape 52 est ensuite terminée et l'étape 5 d'ajustement se poursuit avec la troisième sous-étape 53.
   o Si la réponse est non, on réalise l'étape 520 précédemment décrite, selon laquelle on compare la valeur initiale de cylindre CYL1 et la valeur initiale d'axe de cylindre AX1 avec la valeur totale attendue ATA pour ces deux valeurs. On réalise ensuite un test T8' selon lequel le calculateur répond à la question : « la valeur initiale de cylindre CYL1 présente-t-elle une différence de puissance supérieure ou égale à x' dioptrie avec sa valeur totale attendue ATA, et/ou la valeur initiale d'axe de cylindre AX1 présente-t-elle une différence supérieure ou égale à y'° avec sa valeur totale attendue ATA ? ». Dans le cadre du critère appliqué lors du test T8', la valeur de x' dioptrie appartient préférentiellement à l'intervalle [1 dioptrie ; 2 dioptries] et est plus préférentiellement égale à 1,50 dioptrie. La valeur de y'° appartient préférentiellement à l'intervalle [20° ; 25°] et est plus préférentiellement égale à 20°.

- Si la réponse est non, la deuxième sous-étape 52 et l'étape 5 d'ajustement sont terminées. Le procédé 100 se poursuit avec l'étape 10 précédemment décrite.
- Si la réponse est oui, on réalise l'étape 521 précédemment décrite, selon laquelle on réalise une acquisition d'une valeur ajustée AVA d'acuité visuelle de l'œil, en utilisant la valeur totale attendue ATA pour le cylindre et l'axe de l'optique. On réalise ensuite l'étape f5 précédemment décrite. Dans ce cas particulier, si l'étape 5 d'ajustement ne prend pas fin au cours de l'étape f5, la suite « s5 » de l'étape 5 d'ajustement a lieu et correspond à la troisième sous-étape 53.

La valeur totale attendue ATA pour le cylindre et l'axe est typiquement obtenue en combinant une première composante « cornéenne » comportant une valeur (-CYLK) de cylindre et une valeur AXK d'axe de cylindre, la composante cornéenne étant obtenue grâce à un kératomètre, avec une deuxième composante « interne » ayant une valeur de cylindre de -0,50 dioptrie et une valeur d'axe de cylindre de 90°. La valeur totale attendue ATA pour le cylindre est donc égale à la combinaison du cylindre cornéen (-CYLK)AXK et du cylindre interne estimé à (-0,50 dioptrie) 90°.

Chaque acquisition réalisée lors de l'étape 522 utilise un jeu ajusté de paramètres M2 pour l'optique, ayant :

- une valeur de sphère SPH2 égale à la valeur de sphère de départ SPH1, SPH1', et
- une valeur de cylindre CYL2 égale à la valeur initiale de cylindre CYL1.

Lors de la première acquisition de l'étape 522, la valeur d'axe de cylindre AX2 du jeu ajusté de paramètres M2 présente préférentiellement une variation de +10° par rapport à la valeur initiale d'axe de cylindre AX1 :

$$AX2 = AX1 + 10°$$

L'étape f5 précédemment décrite a ensuite lieu, qui détermine si l'étape 5 d'ajustement continue ou est terminée. Si l'étape 5 d'ajustement continue, une deuxième acquisition de l'étape 522 est réalisée avec une valeur d'axe de cylindre AX2 du jeu ajusté de paramètres M2 qui présente préférentiellement une variation de -10° par rapport à la valeur initiale d'axe de cylindre AX1 :

$$AX2 = AX1 - 10°$$

L'étape f5 a ensuite lieu. Si l'étape 5 d'ajustement n'est pas terminée à l'issue de l'étape f5, l'étape 5 d'ajustement continue avec la troisième sous-étape 53.

Chaque acquisition réalisée lors de l'étape 523 utilise un jeu ajusté de paramètres M2 pour l'œil ayant :

- une valeur de cylindre CYL2 égale à la valeur initiale de cylindre CYL1, et
- une valeur d'axe de cylindre AX2 égale à la valeur initiale d'axe de cylindre AX1.

Lors de la première acquisition de l'étape 523, la valeur de sphère SPH2 du jeu ajusté de paramètres M2 présente préférentiellement une première variation positive par rapport à la valeur de sphère de départ SPH1, SPH1' :

$$SPH2 = (SPH1, SPH1') + k1 \, dioptrie$$

L'étape f5 précédemment décrite a ensuite lieu, qui détermine si l'étape 5 d'ajustement continue ou est terminée. Si l'étape 5 d'ajustement continue, une deuxième acquisition de l'étape 523 est réalisée avec une valeur de sphère SPH2 du jeu ajusté de paramètres M2 qui présente préférentiellement une première variation négative par rapport à la valeur de sphère de départ SPH1, SPH1' :

$$SPH2 = (SPH1, SPH1') - k1 \, dioptrie$$

L'étape f5 a ensuite lieu. Si l'étape 5 d'ajustement continue à l'issue de l'étape f5, une troisième acquisition de l'étape 523 est réalisée avec une valeur de sphère SPH2 du jeu ajusté de paramètres M2 qui présente préférentiellement une deuxième variation positive par rapport à la valeur de sphère de départ SPH1, SPH1' :

$$SPH2 = (SPH1, SPH1') + k2 \, dioptrie$$

L'étape f5 a ensuite lieu. Si l'étape 5 d'ajustement continue à l'issue de l'étape f5, une quatrième acquisition de l'étape 523 est réalisée avec une valeur de sphère SPH2 du jeu ajusté de paramètres M2 qui présente préférentiellement une deuxième variation négative par rapport à la valeur de sphère de départ SPH1, SPH1' :

$$SPH2 = (SPH1, SPH1') - k2 \, dioptrie$$

L'étape f5 a ensuite lieu. Si l'étape 5 d'ajustement n'est pas terminée à l'issue de l'étape f5, l'étape 5 d'ajustement continue avec la troisième sous-étape 53.

Lors de l'étape 523, les valeurs k1 et k2 des première et deuxième variations sont préférentiellement choisies parmi (0,50 ; 1,00 ; 1,50).

Si la deuxième sous-étape 52 s'est terminée avec l'étape 521, la troisième sous-étape 53 comporte l'étape 523 précédemment décrite, selon laquelle on réalise au plus quatre acquisitions de valeur ajustée AVA d'acuité visuelle de l'œil, en faisant varier la valeur de sphère de l'optique par rapport à la valeur de sphère de départ SPH1, SPH1'. La troisième sous-étape 53 et l'étape 5 d'ajustement sont ensuite terminées. Le procédé 100 de bilan intermédiaire se poursuit avec l'étape 10 précédemment décrite.

Si la deuxième sous-étape 52 s'est terminée avec l'étape 522, la troisième sous-étape 53 comporte d'abord l'étape 520 précédemment décrite, selon laquelle on compare la valeur initiale de cylindre CYL1 et la valeur initiale d'axe de cylindre AX1 avec la valeur totale attendue ATA pour ces deux valeurs. On réalise ensuite un test T8", selon lequel le calculateur répond à la question : « la valeur initiale de cylindre CYL1 présente-t-elle une différence de puissance supérieure ou égale à x" dioptrie avec sa valeur totale attendue ATA, et/ou la valeur initiale d'axe de cylindre AX1 présente-t-elle une différence supérieure ou égale à y"° avec sa valeur totale attendue ATA ? ». Dans le cadre du critère appliqué lors du test T8", la valeur de x" dioptrie appartient préférentiellement à l'intervalle [1 dioptrie ; 1,50 dioptrie] et est plus préférentiellement égale à 1,00 dioptrie. La valeur de y"° appartient préférentiellement à l'intervalle [15° ; 20°] et est plus préférentiellement égale à 15°.

- Si la réponse est non, la troisième sous-étape 53 et l'étape 5 d'ajustement sont terminées et le procédé 100 de bilan intermédiaire se poursuit avec l'étape 10 précédemment décrite.
- Si la réponse est oui, on réalise l'étape 521 précédemment décrite, selon laquelle on réalise une acquisition d'une valeur ajustée AVA d'acuité visuelle de l'œil, en utilisant la valeur totale attendue ATA pour le cylindre et l'axe de l'optique. On réalise ensuite l'étape f5 précédemment décrite. Dans ce cas particulier, si l'étape d'ajustement 5 ne prend pas fin au cours de l'étape f5, la suite « s5 » de l'étape 5 d'ajustement a lieu mais correspond également à la fin de l'étape 5 d'ajustement, puis le procédé 100 de bilan intermédiaire se poursuit avec l'étape 10 précédemment décrite.

Si la deuxième étape 52 s'est terminée avec l'étape 523, la troisième sous-étape 53 comporte d'abord l'étape 520 précédemment décrite, selon laquelle on compare la valeur initiale de cylindre CYL1 et la valeur initiale d'axe de cylindre AX1 avec la valeur totale attendue ATA pour ces deux valeurs. On réalise ensuite le test T8' précédemment décrit, selon lequel le calculateur répond à la question : la valeur initiale de cylindre CYL1 présente-t-elle une différence de puissance supérieure ou égale à x' dioptrie avec sa valeur totale attendue, et/ou la valeur initiale d'axe de cylindre AX1 présente-t-elle une différence supérieure ou égale à y'° avec sa valeur totale attendue ATA ?

- Si la réponse est non, la troisième sous-étape 53 et l'étape 5 d'ajustement sont terminées et le procédé 100 de bilan intermédiaire se poursuit avec l'étape 10 précédemment décrite.
- Si la réponse est oui, on réalise l'étape 521 précédemment décrite, selon laquelle on réalise une acquisition d'une valeur ajustée AVA d'acuité visuelle de l'œil, en utilisant la valeur totale attendue ATA pour le cylindre et l'axe de l'optique. On réalise ensuite l'étape f5 précédemment décrite. Dans ce cas particulier, si l'étape 5 d'ajustement ne prend pas fin au cours de l'étape f5, la suite « s5 » de l'étape 5 d'ajustement a lieu mais correspond également à la fin de l'étape 5 d'ajustement, puis le procédé 100 de bilan intermédiaire se poursuit avec l'étape 10 précédemment décrite.

Dans le cadre de la présente invention, on considère préférentiellement que :

- la valeur initiale de cylindre CYL1 est « faible » si elle est telle que:

$$|CYL1| < 1,00 \; dioptrie$$

- la valeur initiale de cylindre CYL1 est « moyenne » si elle est telle que :

$$1,00 \; dioptrie \leq |CYL1| < 3,00 \; dioptries$$

- la valeur initiale de cylindre CYL1 est « forte » si elle est telle que :

$$|CYL1| \geq 3,00 \; dioptries$$

Dans le cadre de la présente invention, lorsque la valeur de sphère de départ SPH1, SPH1' est négative, on considère préférentiellement que :

- la valeur de sphère de départ SPH1, SPH1' est « faible » si elle est telle que :

$$-0,25 \; dioptrie \leq SPH1, SPH1' \leq -2,50 \; dioptries$$

- la valeur de sphère de départ SPH1, SPH1' est « forte » si elle est telle que :

$$SPH1, SPH1' < -2,50 \; dioptries$$

Dans le cadre de la présente invention, lorsque la valeur de sphère de départ SPH1, SPH1' est positive, on considère préférentiellement que :

- la valeur de sphère de départ SPH1, SPH1' est « faible » si elle est telle que :

$$0 \; dioptrie \leq SPH1, SPH1' \leq 2,50 \; dioptries,$$

- la valeur de sphère de départ SPH1, SPH1' est « forte » si elle est telle que :

$$SPH1, SPH1' > 2,50 \; dioptries$$

Dans le cadre de la présente invention et alternativement au paragraphe précédent, lorsque la valeur de sphère de départ SPH1, SPH1' est positive, les valeurs de sphère de départ SPH1, SPH1' définissant les catégories « faible » et « forte » sont préférentiellement choisies en fonction des catégories « faible », « moyenne » ou « forte » de la valeur initiale de cylindre CYL1. Ainsi :

- lorsque la valeur initiale de cylindre CYL1 est « faible », la valeur de sphère de départ SPH1, SPH1' est « faible » si elle est telle que :

$$0 \quad dioptrie \leq SPH, SPH1 \leq +2,50 \; dioptries$$

et la valeur de sphère de départ SPH1, SPH1' est « forte » si elle est telle que :

$$SPH1, SPH1' > +2,50 \; dioptries$$

- Lorsque la valeur initiale de cylindre CYL1 est « moyenne », la valeur de sphère de départ SPH1, SPH1' est « faible » si elle est telle que :

$$0 \; dioptrie \leq SPH, SPH1 \leq +3{,}00 \; dioptries$$

et la valeur de sphère de départ SPH1, SPH1' est « forte » si elle est telle que :

$$SPH1, SPH1' > +3{,}00 \; dioptries$$

- Lorsque la valeur initiale de cylindre CYL1 est « forte », la valeur de sphère de départ SPH1, SPH1' est « faible » si elle est telle que :

$$0 \; dioptrie \leq SPH, SPH1 \leq +2{,}00 \; dioptries,$$

et la valeur de sphère de départ SPH1, SPH1' est « forte » si elle est telle que :

$$SPH1, SPH1' > +2{,}00 \; dioptries$$

Le procédé 100 de bilan intermédiaire qui vient d'être décrit est avantageusement suivi d'un procédé 200 de bilan complet qui est à présent décrit, en lien avec les figures 1, 2c et 3. Le procédé 200 de bilan complet est une mesure de réfraction subjective qui comporte typiquement :

- une étape 7 de test monoculaire qui est réalisée d'abord pour un œil puis pour l'autre œil,
- une étape 8 de test bioculaire qui est réalisée pour les deux yeux ensemble, et
- une étape 9 de test binoculaire qui est réalisée pour les deux yeux ensemble.

La mesure de réfraction subjective peut être réalisée conformément à l'état de la technique, qui est par exemple décrit dans les ouvrages suivants : « Borish's Clinical Refraction (2nd edition) » par William J. Benjamin (2006), et « Primary care Optometry (5th edition) » par Theodore Grosvenor (2007).

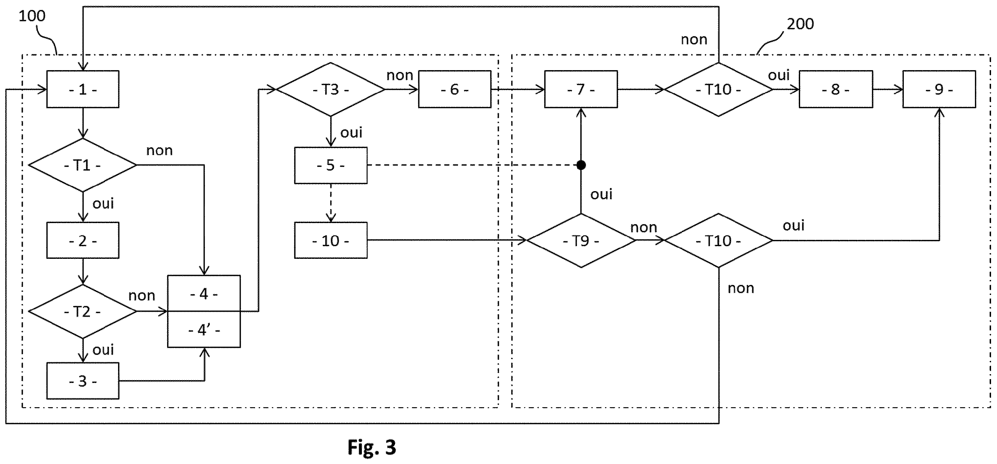

La figure 2c montre que lorsque l'étape f5 met fin à l'étape 5 d'ajustement et au procédé 100 de bilan intermédiaire, elle est avantageusement suivie du procédé 200 de bilan complet. Plus précisément, elle est avantageusement suivie de l'étape 7 de test monoculaire (représentation en trait pointillé sur la figure 3).

La figure 3 montre que lorsque la réponse à la question du test T3, « la valeur modifiée AVM, AVM' d'acuité visuelle de l'œil est-elle strictement inférieure à la valeur de référence AVR d'acuité visuelle de l'œil ? », est « non », le test T3 est avantageusement suivi de l'étape 6 de vérification d'accommodation puis de l'étape 7 de test monoculaire.

La figure 3 montre que l'étape 10, selon laquelle on réalise une acquisition d'une valeur d'acuité visuelle de l'œil en agençant devant l'œil une optique qui est un trou sténopéïque, est avantageusement suivi d'une étape de test T9, selon laquelle le calculateur répond à la question : l'acuité visuelle est-elle meilleure ou identique avec le trou sténopéïque ?

- Si la réponse est oui, on réalise l'étape 7 de test monoculaire.
- Si la réponse est non, on réalise l'étape de test T10 précédemment décrite, selon laquelle le calculateur répond à la question : « le procédé 100 de bilan intermédiaire a-t-il été conduit pour les deux yeux ? ». Si la réponse est « non », le calculateur reboucle sur l'étape 1 du procédé 100 de bilan intermédiaire, pour le deuxième œil. Si la réponse est « oui », le test T10 est suivi de l'étape 9 de test binoculaire.

L'étape 7 de test monoculaire est suivie d'une étape de test T10, selon laquelle le calculateur répond à la question : « le procédé 100 de bilan intermédiaire a-t-il été conduit pour les deux yeux ? ». Si la réponse est « non », le calculateur reboucle sur l'étape 1 du procédé 100 de bilan intermédiaire, pour le deuxième œil. Si la réponse est « oui », le test T10 est suivi de l'étape 8 de test bioculaire, puis de l'étape 9 de test binoculaire.

**Revendications**

1. Procédé (100) de bilan intermédiaire d'un œil d'un utilisateur comportant :

   - une étape (1) d'attribution à l'œil de l'utilisateur d'une valeur de référence AVR d'acuité visuelle, en fonction d'un âge de l'utilisateur et d'un jeu initial de paramètres M1 de l'œil comportant une valeur initiale de sphère SPH1, une valeur initiale de cylindre CYL1 et une valeur initiale d'axe de cylindre AX1, et au moyen d'une matrice ayant une pluralité de L lignes et une pluralité de C colonnes et comportant donc $L \times C$ composantes, chaque ligne correspondant à une catégorie de jeu de paramètres, chaque colonne correspondant à une catégorie d'âge et chaque composante étant associée à une valeur de référence AVR d'acuité visuelle ;
- si (T1) la valeur de sphère SPH1 du jeu initial M1 est telle que :

$$-5,00 \; dioptries \; \leq SPH1 \leq +3,00 \; dioptries,$$

o une étape (2) de mesure d'une valeur brute AVB d'acuité visuelle de l'œil sachant qu'aucune optique n'est placée devant l'œil pour cette mesure
o si (T2) la valeur brute AVB d'acuité visuelle mesurée est supérieure à une valeur brute de référence AVBR d'acuité visuelle, une étape (3) de pondération du jeu initial de paramètres M1 pour l'obtention d'un jeu pondéré de paramètres M1' ayant la valeur initiale de cylindre CYL1, la valeur initiale d'axe de cylindre AX1 et une valeur pondérée de sphère SPH1' telle que :

$$SPH1 + 0,25 \; dioptrie \; \leq SPH1' \leq SPH1 + 0,75 \; dioptrie \; ;$$

- une étape (4, 4') d'acquisition d'une valeur modifiée AVM, AVM' d'acuité visuelle de l'œil, en agençant devant l'œil une optique ayant :

o le jeu pondéré de paramètres M1' si la valeur de sphère SPH1 du jeu initial M1 est telle que -5,00 *dioptries* ≤ *SPH1* ≤ +3,00 *dioptries* ; ou
o le jeu initial de paramètres M1 sinon;

- si (T3) la valeur modifiée AVM, AVM' d'acuité visuelle est strictement inférieure à la valeur de référence AVR d'acuité visuelle, une étape (5) d'ajustement comportant au moins une acquisition d'une valeur ajustée AVA d'acuité visuelle de l'œil, en agençant devant l'œil une optique ayant un jeu ajusté de paramètres M2, le jeu de paramètres M2 étant déterminé à partir :

◦ du jeu pondéré de paramètres M1' si la valeur de sphère SPH1 du jeu initial M1 est telle que -5,00 *dioptries* ≤ *SPH1* ≤ +3,00 *dioptries* ; ou
◦ du jeu de paramètres M1 sinon.

2. Procédé (100) selon la revendication précédente **caractérisé en ce qu'**après chaque acquisition d'une valeur ajustée AVA d'acuité visuelle de l'œil, si (T3') la valeur ajustée AVA d'acuité visuelle mesurée pour l'œil est supérieure ou égale à la valeur de référence AVR d'acuité visuelle, l'étape (5) d'ajustement prend fin.

3. Procédé (100) selon l'une quelconque des revendications précédentes, **caractérisé en ce que** si (T3', T4), avec une optique ayant un jeu de paramètres dont la valeur de sphère présente une variation positive par rapport à la précédente valeur de sphère testée, ou dont la valeur d'axe présente une variation par rapport à la précédente valeur d'axe testée, ou dont les valeurs de cylindre et d'axe correspondent respectivement à une valeur totale attendue ATA, la valeur ajustée AVA d'acuité visuelle acquise pour l'œil est supérieure ou égale à la valeur de référence AVR d'acuité visuelle, alors le procédé comporte une étape (6) selon laquelle on réalise une acquisition d'une valeur d'acuité visuelle de l'œil en augmentant de +0,75 dioptrie la valeur de sphère de l'optique.

4. Procédé (100) selon l'une quelconque des revendications précédentes **caractérisé en ce que** l'étape (5) d'ajustement comporte une première sous-étape (51) dans laquelle on distingue une catégorie de cylindre faible ou moyen et une catégorie de cylindre fort, une catégorie de sphère faible et une catégorie de sphère forte, et selon laquelle :

- si (T5) le cylindre est fort, on réalise (510) au plus quatre acquisitions d'une valeur ajustée AVA d'acuité visuelle de l'œil en faisant varier la valeur d'axe de cylindre de l'optique ;
- si (T5) le cylindre est faible ou moyen et si (T6) la sphère est forte, on réalise (511) au plus quatre acquisitions d'une valeur ajustée AVA d'acuité visuelle de l'œil en faisant varier la valeur de sphère de l'optique ;
- si (T5) le cylindre est faible ou moyen et si (T6) la sphère est faible, on réalise (512) au plus deux acquisitions d'une valeur ajustée AVA d'acuité visuelle de l'œil en faisant varier la valeur de sphère de l'optique.

5. Procédé (100) selon la revendication précédente, **caractérisé en ce que** l'étape d'ajustement (5) comporte une deuxième sous-étape (52) telle que :

- si (T7) le cylindre est faible, on réalise (520) une comparaison du cylindre et de l'axe avec une valeur totale attendue ATA respectivement pour le cylindre et l'axe ; si (T8) le cylindre présente une différence de puissance supérieure ou égale à 0,75 dioptrie avec sa valeur totale attendue ATA et/ou si l'axe présente une différence supérieure ou égale à 25° avec sa valeur totale attendue ATA, alors on réalise (521) une acquisition d'une valeur ajustée AVA d'acuité visuelle de l'œil en utilisant la valeur totale attendue ATA pour le cylindre et l'axe de l'optique ;
- si (T7) le cylindre est moyen, on réalise (522) au plus deux acquisitions d'une valeur ajustée AVA d'acuité visuelle de l'œil en faisant varier la valeur d'axe de l'optique ;
- si (T7) le cylindre est fort et si (T9) la valeur de sphère SPH1 ou de sphère pondérée SPH1' est strictement supérieure à la valeur de cylindre CYL1, on réalise (523) au plus quatre acquisitions de valeur ajustée AVA d'acuité visuelle de l'œil en faisant varier la valeur de sphère de l'optique ;
- si (T7) le cylindre est fort et si (T9) la valeur de sphère SPH1 ou de sphère pondérée SPH1' est inférieure ou égale à la valeur de cylindre CYL1, on réalise (520) une comparaison du cylindre et de l'axe avec la valeur totale attendue ATA pour le cylindre et l'axe ; si (T8') le cylindre présente une différence de puissance supérieure ou égale à 1,50 dioptries avec sa valeur attendue ATA et/ou si l'axe présente une différence supérieure ou égale à 20° avec sa valeur totale attendue ATA, alors on réalise (521) une acquisition d'une valeur ajustée AVA d'acuité visuelle de l'œil en utilisant la valeur totale attendue ATA pour le cylindre et l'axe de l'optique.

6. Procédé (100) selon la revendication précédente **caractérisé en ce que** si (T7) le cylindre est faible, l'étape d'ajustement (5) est terminée à l'issue des première et deuxième sous-étapes (51, 52), sinon l'étape d'ajustement comporte une troisième sous-étape (53) telle que :

- si (T7) le cylindre est moyen, on réalise (520) une comparaison du cylindre et de l'axe avec leur valeur totale attendue ATA ; si (T8") le cylindre présente une différence de puissance supérieure ou égale à 1,00 dioptrie avec sa valeur totale attendue ATA et/ou si l'axe présente une différence supérieure ou égale à 15° avec sa valeur totale attendue ATA, alors on réalise (521) une acquisition d'une valeur ajustée AVA d'acuité visuelle de l'œil en utilisant la valeur totale attendue ATA pour le cylindre et l'axe de l'optique ;
- si (T7) le cylindre est fort et si (T9) la valeur de sphère SPH1 ou de sphère pondérée SPH1' est strictement supérieure à la valeur de cylindre CYL1, on réalise (520) une comparaison du cylindre et de l'axe avec leur valeur totale attendue ATA ; si (T8') le cylindre présente une différence de puissance supérieure ou égale à 1,50 dioptries avec sa valeur totale attendue ATA et/ou si l'axe présente une différence supérieure ou égale à 20° avec sa valeur totale attendue ATA, alors on réalise (521) une acquisition d'une valeur ajustée AVA d'acuité visuelle de l'œil en utilisant la valeur totale attendue ATA pour le cylindre et l'axe de l'optique ;
- si (T7) le cylindre est fort et si (T9) la valeur de sphère SPH1 ou de sphère pondérée SPH1' est inférieure ou égale à la valeur de cylindre CYL1, on réalise (523) au plus quatre acquisitions de valeur ajustée AVA d'acuité visuelle de l'œil en faisant varier la valeur de sphère de l'optique.

7. Produit programme d'ordinateur comportant des instructions logicielles qui, lorsque le programme est exécuté par un ordinateur, mettent en œuvre le procédé selon l'une quelconque des revendications 1 à 6.

8. Support d'enregistrement lisible par un ordinateur, sur lequel est enregistré le produit programme d'ordinateur selon la revendication 7.

9. Dispositif de bilan intermédiaire d'un œil comportant :

- une mémoire de stockage d'une matrice ayant une pluralité de L lignes et une pluralité de C colonnes et comportant donc $L \times C$ composantes, chaque ligne correspondant à une catégorie de jeu de paramètres, chaque colonne correspondant à une catégorie d'âge et chaque composante étant associée à une valeur de référence AVR d'acuité visuelle,
- une mémoire de stockage d'un jeu de paramètres comportant une valeur de sphère, une valeur de cylindre et une valeur d'axe de cylindre,
- une pluralité d'optiques,
- un moyen pour agencer une optique de la pluralité d'optiques devant l'œil,
- un moyen d'affichage,
- un moyen d'acquisition de valeurs d'acuité visuelle de l'œil,
- une mémoire de stockage d'une valeur d'acuité visuelle acquise,
- un calculateur comportant des moyens pour la réalisation des étapes du procédé (100) de bilan intermédiaire d'un œil selon l'une quelconque des revendications 1 à 6.

**Patentansprüche**

1. Verfahren (100) zur Zwischenuntersuchung eines Auges eines Benutzers, umfassend:

   - einen Schritt (1) zum Zuweisen eines AVR-Referenzwerts für die Sehschärfe des Auges des Benutzers in Abhängigkeit vom Alter des Benutzers und einem ursprünglichen Satz von Augenparametern M1, der einen ursprünglichen Sphärenwert SPH1 umfasst, einen ursprünglichen Zylinderwert CYL1 und einen ursprünglichen Zylinderachsenwert AX1, und mittels einer Matrix mit einer Vielzahl von L Zeilen und einer Vielzahl von C Spalten und somit L x C Komponenten, wobei jede Zeile einer Kategorie von Parametersätzen und jede Spalte einer Alterskategorie entspricht und jede Komponente einem AVR-Referenzwert für die Sehschärfe zugeordnet ist;
   - wenn (T1) der Sphärenwert SPH1 des ursprünglichen Satzes M1 derart ist, dass:

   $$-5,00 \ Dioptrien \leq SPH1 \leq +3,00 \ Dioptrien,$$

   o einen Schritt (2) zur Messung eines AVB-Bruttowerts der Sehschärfe des Auges, wobei für diese Messung keine Optik vor dem Auge angeordnet wird;
   o wenn (T2) der gemessene AVB-Bruttowert der Sehschärfe größer ist als ein AVBR-Referenz-Bruttowert der Sehschärfe, einen Schritt (3) zur Gewichtung des ursprünglichen Parametersatzes M1, um einen gewichteten Parametersatz M1' mit dem ursprünglichen Zylinderwert CYL1 zu erhalten, dem ursprünglichen Wert der Zylinderachse AX1 und einem gewichteten Wert der Sphäre SPH1', sodass gilt:

   $$SPH1 + 0,25 \ Dioptrien \leq SPH1' \leq SPH1 + 0,75 \ Dioptrien;$$

   - einen Schritt (4, 4') zum Erfassen eines geänderten Werts AVM, AVM' der Sehschärfe des Auges, indem vor dem Auge eine Optik angeordnet wird, die Folgendes aufweist:

   o den gewichteten Parametersatz M1', wenn der Sphärenwert SPH1 des ursprünglichen Parametersatzes M1 wie folgt ist:

   $$-5,00 \ Dioptrien \leq SPH1 \leq +3,00 \ Dioptrien;$$

   oder
   o andernfalls den ursprünglichen Parametersatz M1

   - wenn (T3) der geänderte AVM/AVM'-Wert der Sehschärfe streng kleiner ist als der AVR-Referenzwert der Sehschärfe, einen Anpassungsschritt (5) mit mindestens einer Erfassung eines angepassten AVA-Wertes der Sehschärfe des Auges, indem vor dem Auge eine Optik mit einem angepassten Parametersatz M2 angeordnet wird, wobei der Parametersatz M2 bestimmt wird aus:

   o dem gewichteten Parametersatz M1', wenn der Sphärenwert SPH1 des ursprünglichen Parametersatzes M1 derart ist, dass

   $$-5,00 \ Dioptrien \leq SPH1 \leq +3,00 \ Dioptrien;$$

   oder
   o andernfalls den Parametersatz M1.

2. Verfahren (100) nach dem vorhergehenden Anspruch, **dadurch gekennzeichnet, dass** nach jeder Erfassung eines angepassten AVA-Wertes der Sehschärfe des Auges, wenn (T3') der angepasste AVA-Wert der für das Auge gemessenen Sehschärfe größer oder gleich dem AVR-Referenzwert der Sehschärfe ist, der Anpassungsschritt (5) beendet wird.

3. Verfahren (100) nach einem der vorhergehenden Ansprüche, **dadurch gekennzeichnet, dass**, wenn (T3', T4) mit einer Optik, deren Parameterwert im Vergleich zum zuvor getesteten Sphärenwert eine positive Abweichung aufweist oder deren Achsenwert eine Abweichung vom zuvor getesteten Achsenwert aufweist oder deren Zylinder- und Achsenwerte jeweils einem erwarteten ATA-Gesamtwert entsprechen, der angepasste AVA-Wert der für das Auge ermittelten Sehschärfe größer oder gleich dem AVR-Referenzwert der Sehschärfe ist, dann umfasst das Verfahren einen Schritt (6), in dem eine Ermittlung eines Sehschärfewertes des Auges durchgeführt wird, indem der Sphärenwert der Optik um +0,75 Dioptrien erhöht wird.

4. Verfahren (100) nach einem der vorhergehenden Ansprüche, **dadurch gekennzeichnet, dass** der Anpassungsschritt (5) einen ersten Teilschritt (51) umfasst, in dem eine Kategorie schwacher oder mittlerer Zylinder, eine Kategorie starker Zylinder, eine Kategorie schwacher Sphären und eine Kategorie starker Sphären unterschieden werden, und wobei

   - wenn (T5) die Zylinderstärke stark ist, höchstens vier Erfassungen eines angepassten AVA-Wertes der Sehschärfe des Auges durchgeführt werden (510), indem der Zylinderachsenwert der Optik variiert wird;
   - wenn (T5) die Zylinderstärke schwach oder mittel ist und wenn (T6) die Sphärenstärke stark ist, höchstens vier Erfassungen eines angepassten AVA-Wertes der Sehschärfe des Auges durchgeführt werden (511), indem der Sphärenwert der Optik variiert wird;
   - wenn (T5) die Zylinderstärke schwach oder mittel ist und wenn (T6) die Sphärenstärke schwach ist, höchstens zwei Erfassungen eines angepassten AVA-Wertes der Sehschärfe des Auges durchgeführt werden (512), indem der Sphärenwert der Optik variiert wird.

5. Verfahren (100) nach dem vorhergehenden Anspruch, **dadurch gekennzeichnet, dass** der Anpassungsschritt (5) einen zweiten Teilschritt (52) umfasst, sodass:

   - wenn (T7) die Zylinderstärke gering ist, ein Vergleich der Zylinderstärke und der Achse mit einem erwarteten Gesamtwert ATA für die Zylinderstärke bzw. die Achse durchgeführt wird (520); wenn (T8) die Zylinderstärke eine Stärkendifferenz von mindestens 0,75 Dioptrien gegenüber seinem erwarteten Gesamtwert ATA aufweist und/oder wenn die Achse eine Differenz von mindestens 25° gegenüber ihrem erwarteten Gesamtwert ATA aufweist, eine Erfassung eines angepassten AVA-Wertes der Sehschärfe des Auges unter Verwendung des erwarteten Gesamtwertes ATA für den Zylinder und die Achse der Optik durchgeführt wird (521);
   - wenn (T7) die Zylinderstärke mittel ist, höchstens zwei Erfassungen eines angepassten AVA-Wertes der Sehschärfe des Auges durchgeführt werden (522), indem der Achsenwert der Optik variiert wird;
   - wenn (T7) die Zylinderstärke stark ist und wenn (T9) der Sphärenwert SPH1 oder der gewichtete Sphärenwert SPH1' streng größer ist als der Wert des Zylinders CYL1, höchstens vier Erfassungen des angepassten AVA-Wertes der Sehschärfe des Auges durchgeführt werden (523), indem der Sphärenwert der Optik variiert wird;
   - wenn (T7) die Zylinderstärke stark ist und wenn (T9) der Sphärenwert SPH1 oder der gewichtete Sphärenwert SPH1' kleiner oder gleich dem Zylinderwert CYL1 ist, ein Vergleich der Zylinderstärke und der Achse mit dem erwarteten Gesamtwert ATA für den Zylinder und die Achse durchgeführt wird (520); wenn (T8') die Zylinderstärke eine Stärkendifferenz von mindestens 1,50 Dioptrien gegenüber seinem erwarteten Wert ATA aufweist und/oder wenn die Achse eine Differenz von mindestens 20° gegenüber ihrem erwarteten Gesamtwert ATA aufweist, ein angepasster Wert AVA für die Sehschärfe des Auges unter Verwendung des erwarteten Gesamtwerts ATA für den Zylinder und die Achse der Optik ermittelt wird (521).

6. Verfahren (100) nach dem vorhergehenden Anspruch, **dadurch gekennzeichnet, dass**, wenn (T7) die Zylinderstärke schwach ist, der Anpassungsschritt (5) nach Abschluss des ersten und zweiten Teilschritts (51, 52) beendet ist, andernfalls umfasst der Anpassungsschritt einen dritten Teilschritt (53), sodass:

   - wenn (T7) die Zylinderstärke mittel ist, ein Vergleich des Zylinders und der Achse mit ihrem erwarteten Gesamtwert ATA durchgeführt wird (520); wenn (T8") die Zylinderstärke eine Abweichung von mindestens 1,00 Dioptrie vom erwarteten Gesamtwert ATA aufweist und/oder wenn die Achse eine Abweichung von mindestens 15° vom erwarteten Gesamtwert ATA aufweist, ein angepasster AVA-Wert für die Sehschärfe des Auges unter Verwendung des erwarteten Gesamtwerts ATA für den Zylinder und die Achse der Optik ermittelt wird (521);
   - wenn (T7) die Zylinderstärke groß ist und wenn (T9) der Sphärenwert SPH1 oder der gewichtete Sphärenwert SPH1' streng größer ist als der Wert der Zylinderstärke CYL1, ein Vergleich der Zylinderstärke und der Achse mit ihrem erwarteten Gesamtwert ATA durchgeführt wird (520); wenn (T8') die Zylinderstärke eine Abweichung von mindestens 1,50 Dioptrien von seinem erwarteten Gesamtwert ATA aufweist und/oder wenn die Achse eine Abweichung von mindestens 20° von ihrem erwarteten Gesamtwert ATA aufweist, ein angepasster AVA-Wert für die Sehschärfe des Auges unter Verwendung des erwarteten Gesamtwerts ATA für den Zylinder und die Achse der Optik ermittelt wird (521);

- wenn (T7) die Zylinderstärke groß ist und wenn (T9) der Sphärenwert SPH1 oder der gewichtete Sphärenwert SPH1' kleiner oder gleich dem Zylinderwert CYL1 ist, höchstens vier Erfassungen eines angepassten Sehschärfewerts AVA des Auges durchgeführt werden (523), indem der Sphärenwert der Optik variiert wird.

7. Computerprogrammprodukt mit Softwareanweisungen, die, wenn das Programm von einem Computer ausgeführt wird, das Verfahren gemäß einem der Ansprüche 1 bis 6 ausführen.

8. Computerlesbares Speichermedium, auf dem das Computerprogrammprodukt gemäß Anspruch 7 gespeichert ist.

9. Vorrichtung zur Zwischenuntersuchung eines Auges, mit:

- einem Speicher zum Speichern einer Matrix mit einer Vielzahl von L Zeilen und einer Vielzahl von C Spalten und somit $L$ x $C$ Komponenten, wobei jede Zeile einer Kategorie von Parametersätzen und jede Spalte einer Alterskategorie entspricht und jede Komponente einem AVR-Referenzwert für die Sehschärfe zugeordnet ist,
- einem Speicher zum Speichern eines Parametersatzes mit einem Sphärenwert, einem Zylinderwert und einem Zylinderachsenwert,
- einer Vielzahl von Optiken,
- einem Mittel zum Anordnen einer Optik aus der Vielzahl von Optiken vor dem Auge,
- einer Anzeigeeinrichtung,
- einer Einrichtung zum Erfassen von Sehschärfewerten des Auges,
- einem Speicher zum Speichern eines erfassten Sehschärfewertes,
- einem Rechner mit Mitteln zur Durchführung der Schritte des Verfahrens (100) zur Zwischenuntersuchung eines Auges gemäß einem der Ansprüche 1 bis 6.

## Claims

1. Method (100) for intermediate assessment of an eye of a user comprising:

- a step (1) of attributing to the eye of the user a reference visual acuity value AVR, as a function of the age of the user and an initial set of parameters M1 of the eye comprising an initial sphere value SPH1, an initial cylinder value CYL1 and an initial cylinder axis value AX1, and by means of a matrix having a plurality of L lines and a plurality of C columns and thus comprising LxC components, each line corresponding to a set of parameters category, each column corresponding to an age category and each component being associated with a reference visual acuity value AVR;
- if (T1) the sphere value SPH1 of the initial set M1 is such that:

$$-5,00 \text{ dioptres} \leq \text{SPH1} \leq +3,00 \text{ dioptres},$$

o a step (2) of measuring a raw visual acuity value AVB of the eye, knowing that no optic is placed in front of the eye for this measuring;
o if (T2) the measured raw visual acuity value AVB is greater than a reference raw visual acuity value AVBR, a step (3) of weighting the initial set of parameters M1 to obtain a weighted set of parameters M1' having the initial cylinder value CYL1, the initial cylinder axis value AX1 and a weighted sphere value SPH1' such that:

$$SPH1 + 0,25 \, dioptre \leq SPH1' \leq SPH1 + 0,75 \, dioptre \, ;$$

- a step (4, 4') of acquiring a modified visual acuity value AVM, AVM' of the eye, by arranging in front of the eye an optic having:

o the weighted set of parameters M1' if the sphere value SPH1 of the initial set M1 is such that -5,00 $dioptre \leq$

$$SPH1 \leq +3,00 \, dioptre \, ;$$

or
o the initial set of parameters M1 otherwise;

- if (T3) the modified visual acuity value AVM, AVM' is strictly less than the reference visual acuity value AVR, a step (5) of adjustment comprising at least one acquisition of an adjusted visual acuity value AVA of the eye, by arranging in front of the eye an optic having an adjusted set of parameters M2, the adjusted set of parameters M2 being determined from:

o the weighted set of parameters M1' if the sphere value SPH1 of the initial set M1 is such that -5,00 *dioptre* ≤ *SPH1* ≤ +3,00 *dioptre* ; or
o the set of parameters M1 otherwise.

2. Method (100) according to the preceding claim **characterised in that** after each acquisition of an adjusted visual acuity value AVA of the eye, if (T3') the adjusted visual acuity value AVA measured for the eye is greater than or equal to the reference visual acuity value AVR, the step of adjustment (5) ends.

3. Method (100) according to any of the preceding claims, **characterised in that** if (T3', T4), with an optic having a set of parameters of which the sphere value has a positive variation compared to the preceding tested sphere value, or of which the axis value has a variation compared to the preceding tested axis value, or of which the cylinder and axis values correspond respectively to an expected total value ATA, the adjusted visual acuity value AVA acquired for the eye is greater than or equal to the reference visual acuity value AVR, then the method comprises a step (6) according to which an acquisition is made of a visual acuity value of the eye by increasing by +0.75 dioptre the sphere value of the optic.

4. Method (100) according to any of the preceding claims **characterised in that** the step of adjustment (5) comprises a first sub-step (51) in which a low or medium cylinder category and a high cylinder category, a low sphere category and a high sphere category are distinguished, and according to which:

- if (T5) the cylinder is high, at the most four acquisitions are made (510) of an adjusted visual acuity value AVA of the eye by varying the cylinder axis value of the optic;
- if (T5) the cylinder is low or medium and if (T6) the sphere is high, at the most four acquisitions are made (511) of an adjusted visual acuity value AVA of the eye by varying the sphere value of the optic;
- if (T5) the cylinder is low or medium and if (T6) the sphere is low, at the most two acquisitions are made (512) of an adjusted visual acuity value AVA of the eye by varying the sphere value of the optic.

5. Method (100) according to the preceding claim, **characterised in that** the step of adjustment (5) comprises a second sub-step (52) such that:

- if (T7) the cylinder is low, (520) a comparison is made (520) of the cylinder and the axis with an expected total value ATA respectively for the cylinder and the axis; if (T8) the cylinder has a power difference greater than or equal to 0.75 dioptre with its expected total value ATA and/or if the axis has a difference greater than or equal to 25° with its expected total value ATA, then an acquisition is made (521) of an adjusted visual acuity value AVA of the eye using the expected total value ATA for the cylinder and the axis of the optic;
- if (T7) the cylinder is medium, at the most two acquisitions are made (522) of an adjusted visual acuity value AVA of the eye by varying the axis value of the optic;
- if (T7) the cylinder is high and if (T9) the sphere value SPH1 or weighted sphere value SPH1' is strictly greater than the cylinder value CYL1, at the most four acquisitions are made (523) of an adjusted visual acuity value AVA of the eye by varying the sphere value of the optic;
- if (T7) the cylinder is high and if (T9) the sphere value SPH1 or weighted sphere value SPH1' is less than or equal to the cylinder value CYL1, a comparison is made (520) of the cylinder and the axis with the expected total value ATA for the cylinder and the axis; if (T8') the cylinder has a power difference greater than or equal to 1.50 dioptres with its expected value ATA and/or if the axis has a difference greater than or equal to 20° with its expected total value ATA, then an acquisition is made (521) of an adjusted visual acuity value AVA of the eye using the expected total value ATA for the cylinder and the axis of the optic.

6. Method (100) according to the preceding claim **characterised in that** if (T7) the cylinder is low, the step of adjustment (5) is terminated at the end of the first and second sub-steps (51, 52), if not the step of adjustment comprises a third sub-step (53) such that:

- if (T7) the cylinder is medium, a comparison is made (520) of the cylinder and the axis with their expected total value ATA; if (T8") the cylinder has a power difference greater than or equal to 1,00 dioptre with its expected total value ATA and/or if the axis has a difference greater than or equal to 15° with its expected total value ATA, then an acquisition is made (521) of an adjusted visual acuity value AVA of the eye using the expected total value ATA for the cylinder and the axis of the optic;

- if (T7) the cylinder is high and if (T9) the sphere value SPH1 or weighted sphere value SPH1' is strictly greater than the cylinder value CYL1, a comparison is made (520) of the cylinder and the axis with their expected total value ATA; if (T8') the cylinder has a power difference greater than or equal to 1.50 dioptres with its expected total value ATA and/or if the axis has a difference greater than or equal to 20° with its expected total value ATA, then an acquisition is made (521) of an adjusted visual acuity value AVA of the eye using the expected total value ATA for the cylinder and the axis of the optic;

- if (T7) the cylinder is high and if (T9) the sphere value SPH1 or weighted sphere value SPH1' is less than or equal to the cylinder value CYL1, at the most four acquisitions are made (523) of an adjusted visual acuity value AVA of the eye by varying the sphere value of the optic.

7. Computer program product comprising software instructions which, when the programme is executed by a computer, implement the method according to any of claims 1 to 6.

8. Recording support readable by a computer, on which is recorded the computer programme product according to claim 7.

9. Device for intermediate assessment of an eye comprising:

- a memory for storing a matrix having a plurality of L lines and a plurality of C columns and thus comprising LxC components, each line corresponding to a set of parameters category, each column corresponding to an age category and each component being associated with a reference visual acuity value AVR,
- a memory for storing a set of parameters comprising a sphere value, a cylinder value and a cylinder axis value,
- a plurality of optics,
- a means for arranging an optic of the plurality of optics in front of the eye,
- a display means,
- a means for acquiring visual acuity values of the eye,
- a memory for storing an acquired visual acuity value,
- a calculator comprising means for carrying out the steps of the method (100) for intermediate assessment of an eye according to any of claims 1 to 6.

**Fig. 1**

**Fig. 2c**

**Fig. 2a**

**Fig. 2b**

Fig. 3

**EP 3 544 484 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5914772 A

**Littérature non-brevet citée dans la description**

- **WILLIAM J. BENJAMIN**. Borish's Clinical Refraction (2nd edition). 2006
- **THEODORE GROSVENOR**. Primary care Optometry (5th edition). 2007